# FASCICULE DE BREVET EUROPEEN

(11) **EP 2 361 378 B1**
(45) Date de publication et mention de la délivrance du brevet: **27.04.2016**
(21) Numéro de dépôt: 09783980.7
(22) Date de dépôt: 13.10.2009
(51) Int. Cl.: G01N 27/00, G01N 29/02, C23C 16/27, G01N 33/543

(54) **Capteur de type gravimétrique ayant une couche sensible à base de nano-poudre de diamant**
Gravimetrischer Sensor mit einer ein Diamant-nanopulver enthaltenden sensitiven Schicht
Gravimetric sensor having a sensitive layer containing a diamond nanopowder

(30) Priorité: 15.10.2008 FR 0857000
(43) Date de publication de la demande: 31.08.2011
(73) Titulaire: COMMISSARIAT A L'ENERGIE ATOMIQUE ET AUX ENERGIES ALTERNATIVES, 75015 Paris (FR)
(72) Inventeur: SCORSONE, Emmanuel, F-78114 Magny-les-Hameaux (FR)
(74) Mandataire: Ilgart, Jean-Christophe
(86) Numéro de dépôt international: PCT/EP2009/063347
(87) Numéro de publication internationale: WO 2010/043615

(56) Documents cités:
- EP-A- 1 926 211
- HUANG T S ET AL: "Immobilization of antibodies and bacterial binding on nanodiamond and carbon nanotubes for biosensor applications" DIAMOND AND RELATED MATERIALS, ELSEVIER SCIENCE PUBLISHERS, AMSTERDAM, NL, vol. 13, no. 4-8, 1 avril 2004 (2004-04-01), pages 1098-1102, XP004507925 ISSN: 0925-9635
- CHANIOTAKIS N ET AL: "Novel semiconductor materials for the development of chemical sensors and biosensors: A review" ANALYTICA CHIMICA ACTA, ELSEVIER, AMSTERDAM, NL, vol. 615, no. 1, 12 mai 2008 (2008-05-12), pages 1-9, XP022623081 ISSN: 0003-2670 [extrait le 2008-03-30]
- EL HAKIKI ET AL: "Diamond film on Langasite substrate for surface acoustic wave devices operating in high frequency and high temperature" DIAMOND AND RELATED MATERIALS, ELSEVIER SCIENCE PUBLISHERS, AMSTERDAM, NL, vol. 16, no. 4-7, 13 avril 2007 (2007-04-13), pages 966-969, XP022049750 ISSN: 0925-9635
- LUO J K ET AL: "Diamond and diamond-like carbon MEMS" JOURNAL OF MICROMECHANICS & MICROENGINEERING, INSTITUTE OF PHYSICS PUBLISHING, BRISTOL, GB, vol. 17, no. 7, 1 juillet 2007 (2007-07-01), pages S147-S163, XP020120165 ISSN: 0960-1317

## Description

### DOMAINE TECHNIQUE

L'invention a trait à un capteur gravimétrique de type résonateur acoustique permettant de détecter des espèces chimiques ou biochimiques grâce à la présence d'une couche sensible en nano-poudre de diamant.

### ÉTAT DE LA TECHNIQUE ANTÉRIEURE

Le fonctionnement des capteurs chimiques ou biochimiques gravimétriques de type résonateur acoustique est basé sur la mesure d'une variation de la fréquence de résonance du capteur ou plus rarement sur une mesure de déphasage. En fait, ces capteurs sont obtenus en recouvrant une face d'un transducteur gravimétrique du type résonateur acoustique (également appelé transducteur acoustique, par exemple un transducteur piézoélectrique) avec une couche sensible préparée à base d'un matériau capable d'adsorber, de façon plus ou moins sélective, les espèces chimiques ou biochimiques à détecter. Quand une ou plusieurs espèces à détecter interagit avec la couche sensible, la masse de la couche sensible augmente, ce qui entraine une décroissance de la fréquence de résonance du transducteur. La masse adsorbée sur la couche sensible induit donc un changement de la fréquence de résonance du transducteur, qui peut être mesurée. Dans certains cas, c'est le décalage de phase du résonateur acoustique qui est utilisé pour la mesure.

L'inconvénient de tels capteurs est que toute autre variation des propriétés acoustiques à la surface du capteur, telles que des variations des paramètres intrinsèques du matériau sensible (comme sa densité ou son module élastique) ou encore de l'épaisseur du matériau sensible contribuent aussi, dans certains cas de façon non négligeable, à faire varier la fréquence de résonance du transducteur et ainsi à fausser les mesures obtenues par le capteur.

Les couches sensibles déposées sur ces transducteurs sont généralement choisies pour leur affinité la plus spécifique possible avec les molécules ou les espèces biologiques à détecter.

Les matériaux les plus fréquemment utilisés pour constituer la couche sensible sont des polymères. Le polymère utilisé est généralement choisi pour son caractère physicochimique complémentaire avec celui de l'espèce chimique ou biochimique à détecter. Ainsi, des composés volatils comportant une fonction acide permettant la formation d'une liaison hydrogène auront une affinité accrue avec un polymère comportant une fonction basique permettant, elle aussi, la formation d'une liaison hydrogène.

L'inconvénient de ces matériaux polymères est que leur utilisation sur des transducteurs acoustiques nécessite un contrôle strict des propriétés de la couche sensible telles que l'épaisseur, l'uniformité, la viscosité et l'adhérence de la couche sensible, afin d'obtenir des performances suffisantes et reproductibles. Or, les méthodes de dépôt pouvant potentiellement satisfaire ces conditions, comme la méthode Langmuir-Blodgett, l'électropolymérisation *in situ* ou la méthode des monocouches auto-assemblées, sont limitées à quelques systèmes polymère / surface ayant des propriétés spécifiques. On peut lire à ce sujet les documents **[1]** et **[2]** référencés à la fin de cette description.

Les techniques de dépôt pouvant être employées avec un grand nombre de polymères, telles que la vaporisation, le dépôt par tournette ou encore le dépôt de gouttes d'un polymère dissous dans un solvant, ne permettent pas aujourd'hui d'obtenir des dépôts ayant une reproductibilité et une uniformité suffisantes. En effet, les défauts observés sur les couches ainsi obtenues sont bien connus pour dégrader les performances des capteurs obtenus par dépôt d'une couche sensible sur un transducteur acoustique (voir le document **[3]**).

D'autre part, afin de rendre les films polymères plus sélectifs vis-à-vis des espèces chimiques ou biochimiques à détecter, des polymères à empreinte moléculaire ont été développés. Ces polymères sont des polymères hautement réticulés à l'intérieur desquels des cavités spécifiques ont été crées pour piéger une molécule cible spécifique. De tels matériaux ont par exemple été testés sur des capteurs de type résonateur pour la détection du TNT (voir par exemple le document **[4]**) ou encore pour la détection du diméthylméthyl-phosphonate (DMMP) (document **[5]**). Cependant, les polymères à empreinte moléculaire possèdent généralement moins de sites d'interaction avec les molécules cibles que les polymères décrits précédemment. Ainsi, pour obtenir une sensibilité suffisante, il faut déposer sur le capteur du type résonateur une quantité importante de polymère, ce qui peut affecter les performances acoustiques du capteur. De plus, les polymères à empreinte moléculaire actuels restent appliqués à la détection d'un nombre limité de composés pour lesquels une empreinte moléculaire est réalisable dans une matrice polymère.

Une autre famille de matériaux adsorbants prometteurs pour leur utilisation en tant que couche sensible sont les complexes métalliques de macrocycles, tels que les phthalocyanines ou encore les porphyrines. Ces molécules sont des composés stables dont les propriétés peuvent être finement ajustées par la simple modification de leur structure moléculaire de base. En particulier, il a été démontré que la sélectivité chimique de ces composés dépend à la fois du métal de coordination du complexe métallique et de ses groupes fonctionnels de substitution périphériques (voir le document **[6]**). Cependant, la morphologie des films réalisés à partir de complexes métalliques de macrocycles influence aussi les performances de détection des macrocycles. En effet, le caractère aromatique de ces composés engendre des interactions π - π entre les macrocycles, ce qui résulte en des films très compacts avec des coefficients de diffusion faible et donc un temps de réponse du capteur long et une sensibilité moins importante (voir le document **[7]**).

Plus récemment, les nanotubes de carbone sont aussi apparus comme étant des matériaux adsorbants intéressants pour leur utilisation sur des capteurs du type gravimétrique. En effet, les nanotubes de carbone possèdent une structure tubulaire offrant un rapport surface / volume important, une grande mobilité électronique, ainsi qu'une réactivité chimique importante avec de nombreuses possibilités de fonctionnalisation sur leur surface carbonée. Cependant, il est difficile d'obtenir un dépôt uniforme et reproductible de nanotubes de carbone. En effet, de par leur structure allongée, les nanotubes de carbone sont relativement difficiles à disperser. L'obtention d'un dépôt uniforme nécessite souvent l'ajout de surfactant dans la solution de préparation ou d'une matrice organique telle que l'arachidate de cadmium lorsque le dépôt est réalisé par la méthode de Langmuir-Blodgett (voir par exemple le document **[8]**). Or ces additifs ou résidus organiques peuvent persister dans la couche de nanotubes de carbone, même après rinçage de la couche, et peuvent altérer la sélectivité du capteur.

Il est également possible de déposer des couches d'oxydes métalliques sur des capteurs résonateurs acoustiques pour réaliser la détection de gaz réducteurs tels que le dihydrogène. Le trioxyde de tungstène semble se distinguer comme étant un des matériaux les plus prometteurs pour cette application (voir le document **[9]**). Cependant, ce type de couche sensible est réservé à la détection de quelques gaz réducteurs, comme NH₃, 0₃, N0ₓ, H₂S et H₂.

Enfin, il est connu qu'une couche de diamant peut être utilisée comme couche sensible (voir le document **[10]**). Le diamant est un matériau particulièrement appréciable car il est extrêmement stable chimiquement et possède une fenêtre électrochimique large. Cependant, une couche de diamant ne peut être déposée que sur quelques matériaux piézoélectriques qui peuvent supporter les températures élevées nécessaires à la formation par dépôt chimique en phase vapeur de la couche de diamant.

### EXPOSÉ DE L'INVENTION

Comme nous venons de le voir, les couches sensibles proposées dans l'art antérieur présentent des inconvénients. L'inventeur s'est donc fixé comme objectif d'élaborer une couche sensible qui puisse être déposée sur un transducteur gravimétrique du type résonateur acoustique de manière uniforme et reproductible, et qui puisse adsorber de nombreuses sortes d'espèces chimiques ou biochimiques.

Cet objectif est atteint par un capteur chimique ou biochimique gravimétrique du type résonateur acoustique, comprenant :
- un transducteur gravimétrique du type résonateur acoustique ;
- une couche, appelée couche sensible, apte à adsorber une ou plusieurs espèces chimiques ou biochimiques déterminées, ladite couche sensible étant formée sur une face du transducteur gravimétrique du type résonateur acoustique,
caractérisé en ce que la couche sensible comprend une poudre de diamant dont les grains sont de taille nanométrique et forment un empilement tridimensionnel.

Dans ce qui précède et ce qui suit, le terme « taille », appliqué à des grains, désigne la plus grande dimension de ces grains, et le terme « nanométrique » signifie supérieur ou égal à 1 nanomètre et inférieur à 1000 nanomètres.

Pour déterminer la taille des grains de diamant, on peut par exemple utiliser la méthode de diffusion dymanique de la lumiere à travers une dispersion des poudres de diamant dans un solvant approprié.

On rappelle par ailleurs que le terme « grains » désigne des particules dont le rapport de la plus grande dimension sur la plus petite dimension est inférieur ou égal à 5. Les grains peuvent donc être sphériques ou quasi-sphériques, cubiques ou quasi-cubiques, rhomboédrique ou quasi-rhomboédrique...

Dans ce qui suit, on pourra utiliser le terme « nano-poudre de diamant » à la place de l'expression « poudre de diamant dont les grains sont de taille nanométrique », étant entendu que la « nano-poudre » désigne un ensemble de grains dont la plus grande dimension est supérieure ou égale à 1 nanomètre et inférieure à 1000 nanomètres.

On obtient une couche comprenant de la poudre de diamant, c'est-à-dire des grains qui sont dissociés les uns par rapport aux autres. Comme les grains sont de taille nanométrique, la couche garde sa cohésion grâce aux forces de cohésion chimique de type Van der Walls s'établissant entre les grains.

De préférence, la couche sensible est constituée uniquement de la poudre de diamant dont les grains sont de taille nanométrique.

Avantageusement, le transducteur gravimétrique du type résonateur acoustique est un transducteur piézoélectrique.

La structure d'un transducteur piézoélectrique est connue de l'homme du métier. Un transducteur piézoélectrique comprend une couche (ou un substrat) de matériau piézoélectrique, une première électrode et une seconde électrode conductrices formées sur une même face ou sur deux faces distinctes de ladite couche de matériau piézoélectrique. La couche sensible, en étant formée sur une face du transducteur piézoélectrique, va être formée sur la face de la couche de matériau piézoélectrique comprenant la première et/ou la seconde électrode(s) conductrice(s).

Selon une première variante, le transducteur piézoélectrique est un transducteur à onde acoustique de surface (SAW).

Selon une seconde variante, le transducteur piézoélectrique est un transducteur à microbalance à quartz (MBQ).

Selon une troisième variante, le transducteur piézoélectrique est un transducteur choisi parmi un transducteur de type « Dispositifs à ondes de flexion » (FPW pour « Flexural Plate Wave » en anglais), un transducteur à ondes acoustiques de volume (FBAR pour « Film Bulk Acoustic Resonator » en anglais) ou un transducteur de type résonateur à onde acoustique de volume en mode harmonique élevé (HBAR pour « High-tone Bulk Acoustic Resonator » en anglais).

Avantageusement, le transducteur gravimétrique du type résonateur acoustique est un transducteur à micro-levier.

Il est à noter que le fonctionnement de ces différents transducteurs acoustiques (SAW, MBQ...) est décrit de façon intensive dans la littérature et est connu de l'homme du métier.

De préférence, la taille des grains est comprise entre 1 nm et 200 nm.

Avantageusement, les grains de la poudre de diamant ont un diamètre compris entre 1 nm et 50 nm.

Avantageusement, les grains de la poudre de diamant ont un diamètre compris entre 1 nm et 15 nm, de préférence entre 5 nm et 15 nm.

Avantageusement, les grains de la poudre de diamant sont oxydés en surface.

Avantageusement, des molécules ayant des groupements fonctionnels aptes à réagir spécifiquement avec un groupement fonctionnel des espèces chimiques ou biochimiques devant être captées sont greffées en surface des grains de la poudre de diamant. De préférence, les molécules greffées en surface des grains de la poudre de diamant sont des macromolécules choisies parmi les porphyrines, les phtalocyanines, les corroles ou des complexes métalliques de celles-ci.

L'invention concerne également un dispositif de détection d'espèces chimiques ou biochimiques comprenant au moins deux capteurs chimiques ou biochimiques gravimétriques du type résonateur acoustique tels que définis ci-dessus dans le cas particulier où les grains de poudre de diamant comprennent des molécules greffées à leur surface. Dans ce cas, les groupements fonctionnels des molécules greffées en surface des grains de la poudre de diamant d'une première couche sensible peuvent être différents des groupements fonctionnels des molécules greffées en surface des grains de la poudre de diamant d'une seconde couche sensible parmi les couches sensibles desdits au moins deux capteurs chimiques ou biochimiques gravimétriques du type résonateur acoustique.

Les dispositifs définis ci-dessus permettent de réaliser des multicapteurs. Par exemple, de tels dispositifs peuvent être, par exemple, un nez électronique.

L'invention concerne également l'utilisation d'une couche de poudre de diamant dont les grains sont de taille nanométrique comme couche sensible à déposer sur un transducteur gravimétrique du type résonateur acoustique pour former un capteur chimique ou biochimique gravimétrique du type résonateur acoustique, tel que décrit ci-dessus.

Avantageusement, la taille des grains est comprise entre 1 nm et 200 nm.

Enfin, l'invention concerne un procédé de réalisation d'un capteur chimique ou biochimique gravimétrique du type résonateur acoustique tel que décrit ci-dessus et comprenant les étapes suivantes :
a) fourniture d'une solution comprenant de la poudre de diamant dispersée, dont les grains sont de taille nanométrique ;
b) formation d'une couche de poudre de diamant sur une face du transducteur gravimétrique de type résonateur acoustique en utilisant la solution fournie à l'étape a).

Selon un premier mode de réalisation, la solution de l'étape a) est obtenue en dispersant de la poudre de diamant dont les grains sont de taille nanométrique dans de l'eau ou de l'éthanol.

Selon un second mode de réalisation, la solution de l'étape a) est obtenue en oxydant la surface des grains d'une poudre de diamant, les grains étant de taille nanométrique, et en dispersant lesdits grains dans de l'eau ou de l'éthanol.

Selon un troisième mode de réalisation, la solution de l'étape a) est obtenue en réalisant les étapes suivantes :
- oxydation de la surface des grains d'une poudre de diamant, les grains étant de taille nanométrique ;
- formation d'une solution par dispersion desdits grains oxydés dans un solvant adéquat, préférentiellement de l'eau ou de l'éthanol ;
- ajout, dans la solution, de réactifs chimiques permettant le greffage préférentiellement covalent de groupements fonctionnels sur les grains oxydés de la poudre de diamant.

Selon un quatrième mode de réalisation, la solution de l'étape a) est obtenue en réalisant les étapes suivantes :
- hydrogénation de la surface des grains d'une poudre de diamant, les grains étant de taille nanométrique ;
- formation d'une solution par mise en solution desdits grains hydrogénés dans un solvant adéquat pour les réactions futures de greffage ;
- ajout, dans la solution, de réactifs chimiques permettant le greffage préférentiellement covalent de groupements fonctionnels sur les grains hydrogénés de la poudre de diamant, et le greffage permettant une dispersion des grains initialement hydrogénés dans la solution.

Il est à noter que la poudre de diamant hydrogénée est très insoluble dans la plupart des solvants, et en particulier dans l'éthanol ou l'eau. Par contre, quand elle est placée dans un solvant adéquat, elle peut se disperser dans le solvant au fur et à mesure que des groupements fonctionnels sont greffés sur sa surface.

Dans les troisième et quatrième modes de réalisation décrits ci-dessus, on précise que le greffage des groupements fonctionnels peut se faire en une ou plusieurs étapes successives en termes de réactions chimiques.

Avantageusement, la taille des grains est comprise entre 1 nm et 200 nm.

Selon un premier mode de réalisation, l'étape b) est obtenue par trempage du transducteur gravimétrique du type résonateur acoustique dans la solution fournie à l'étape a), puis séchage du transducteur sous flux d'un gaz inerte.

Selon un second mode de réalisation, l'étape b) est obtenue par dépôt à la tournette de la solution fournie à l'étape a) sur une face du transducteur gravimétrique du type résonateur acoustique, puis séchage du transducteur sous flux d'un gaz inerte.

Selon un troisième mode de réalisation, l'étape b) est obtenue par pulvérisation de la solution fournie à l'étape a) sur une face du transducteur gravimétrique du type résonateur acoustique, puis séchage du transducteur sous flux d'un gaz inerte.

Dans les trois modes de réalisation ci-dessus, le gaz inerte peut par exemple être de l'argon.

### BRÈVE DESCRIPTION DES DESSINS

L'invention sera mieux comprise et d'autres avantages et particularités apparaîtront à la lecture de la description qui va suivre, donnée à titre d'exemple non limitatif, et accompagnée des dessins annexés parmi lesquels :
- la figure 1 représente un grain de nano-poudre de diamant greffé par un sel de diazinium ;
- la figure 2 représente, selon une vue en coupe, une couche sensible à base de nano-poudre de diamant selon l'invention, la couche sensible étant fonctionnalisée et disposée sur la surface d'un transducteur acoustique (dont seule la surface est représentée) ;
- la figure 3 représente une vue de dessus d'un capteur de type SAW comportant une couche sensible à base de nano-poudre de diamant selon l'invention ;
- la figure 4 représente une vue en coupe d'un capteur de type SAW comportant une couche sensible à base de nano-poudre de diamant selon l'invention ;
- la figure 5 est un graphique représentant les réponses successives à trois expositions de vapeur de xylène d'un capteur SAW ne comportant pas de couche sensible et d'un capteur SAW comportant une couche de nano-poudre de diamant selon l'invention ;
- la figure 6 est un graphique représentant une mesure comparative de la réponse de deux capteurs SAW fonctionnalisés avec des nano-poudres de diamant comportant des terminaisons de surface différentes, lors d'une exposition à des vapeurs chimiques de xylène, puis de DMMP ;
- la figure 7 représente une vue en coupe d'un capteur de type microbalance à quartz comportant une couche sensible à base de nano-poudre de diamant selon l'invention.

### EXPOSÉ DÉTAILLÉ DE MODES DE RÉALISATION PARTICULIERS

L'invention consiste à utiliser de la nano-poudre de diamant comme matériau sensible déposé sur un transducteur gravimétrique de type résonateur acoustique pour réaliser un capteur chimique ou biochimique.

Il est important que les grains de la poudre de diamant soient, d'une part, des particules dont le rapport entre la plus grande dimension et la plus petite dimension est inférieur ou égal à 5 et, d'autre part, qu'ils aient des dimensions nanométriques, c'est-à-dire inférieures à 1000 nm et supérieures ou égales à 1 nm. De préférence, les grains sont sphériques ou quasi-sphériques et ont des dimensions comprises entre 200 nm et 1 nm. En effet, plus les grains de la poudre de diamant sont fins, plus les dépôts réalisés à partir de cette poudre sont homogènes, plus la surface disponible pour capter les molécules est importante et au final, plus la sensibilité du capteur est grande.

La nano-poudre de diamant utilisée est préférentiellement de la poudre de détonation (poudre obtenue par détonation, par exemple à l'aide de TNT (trinitrotoluène)), du fait de la petitesse de sa taille (5 à 15 nanomètres pour la nano-poudre primaire).

Alternativement, la nano-poudre peut aussi être obtenue par broyage de poudre de diamant de taille plus grossière. Le diamètre d'intérêt de la nano-poudre de diamant sera typiquement de l'ordre de 1 à 50 nm.

Dans les deux cas, le coeur de la nano-poudre est composé en grande majorité de carbone hybridé SP3, non dopé et donc intrinsèquement isolant.

La nano-poudre de diamant peut être utilisée sous trois formes possibles, à savoir sous une forme brute (non traitée), sous une forme traitée en surface dans le but de rendre la poudre plus homogène et reproductible, et éventuellement pour la rendre partiellement sélective, et sous une forme greffée avec des fonctions chimiques ou biologiques spécifiquement choisies pour que la nano-poudre ait une affinité particulièrement élevée avec les analytes (espèces chimiques ou biochimiques) à détecter.

Ainsi, la nano-poudre de diamant peut être utilisée brute, si besoin après purification, comme dans le cas de la poudre de détonation.

Elle peut également être traités physiquement ou chimiquement afin d'en modifier la terminaison chimique de surface. Quand la nano-poudre de diamant n'est pas traitée, les atomes de carbone en surface de la nano-poudre sont généralement terminés par des fonctions diverses, comme par exemple des groupements hydroxyles, carboxyliques, et/ou des ponts éthers. Le taux de recouvrement des différentes terminaisons peut varier considérablement en fonction de l'origine de la nano-poudre. Afin d'obtenir une surface plus homogène et reproductible, la nano-poudre de diamant peut être oxydée en surface, soit par exposition à un plasma gazeux contenant une proportion adéquate d'oxygène, soit en procédant à un recuit à haute température dans une atmosphère contrôlée contenant aussi de l'oxygène, soit par voie liquide dans une solution très oxydante. La solution oxydante utilisée sera typiquement une solution acide ou un mélange d'acides (par exemple acide sulfurique H₂SO₄ et acide nitrique HNO₃), un mélange piranha (eau oxygénée et acide sulfurique, H₂O₂ + H₂SO₄) ou toute autre solution oxydante couramment utilisée par l'homme du métier pour le traitement de surfaces en diamant (acide sulfochromique, espèces oxydantes en milieux acides bouillants...). La nano-poudre ainsi oxydée pourra alors être déposé après traitement sur le transducteur acoustique.

Enfin, des groupements fonctionnels peuvent être greffés sur la surface de la nano-poudre de diamant afin de rendre les interactions entre la nano-poudre de diamant et le composé chimique ou biochimique à détecter plus sélectives. Les groupements greffés sont généralement choisis de manière à avoir un caractère physico-chimique complémentaire avec celui du composé à détecter. Par exemple, il pourra s'agir de groupements acides ou basiques permettant une liaison hydrogène avec le composé à détecter, ou de groupements polaires permettant une interaction électrostatique avec le composé à détecter.

Alternativement, les groupements chimiques greffés peuvent être des macromolécules appartenant à la famille des porphyrines, des phthalocyanines ou des corroles, et plus particulièrement des complexes métalliques de ces mêmes composés.

Les techniques de greffage consistent généralement à créer une liaison covalente entre les atomes de carbone en surface du diamant avec un ou plusieurs atomes de la molécule à greffer. Généralement, le greffage se fait sur une surface de diamant traitée. Le traitement consiste à oxyder la surface avec l'une des méthodes décrites précédemment. Alternativement, la nano-poudre de diamant peut être hydrogénée en surface dans un plasma gazeux d'hydrogène. Ensuite, un précurseur est greffé sur la surface traitée. Ce précurseur sera par exemple un sel de diazonium, un silane, ou encore une aminoalkane insaturé (par exemple le 10-aminodéc-1-ène). Le groupement chimique ou biologique comportant un caractère physico-chimique complémentaire avec celui de l'analyte à détecter est ensuite lié à ce précurseur greffé sur la surface du grain de nano-poudre de diamant. Ainsi, la chimie du carbone offre de nombreuses possibilités de greffage de groupements chimiques ou biologiques divers sur la surface de la nano-poudre de diamant.

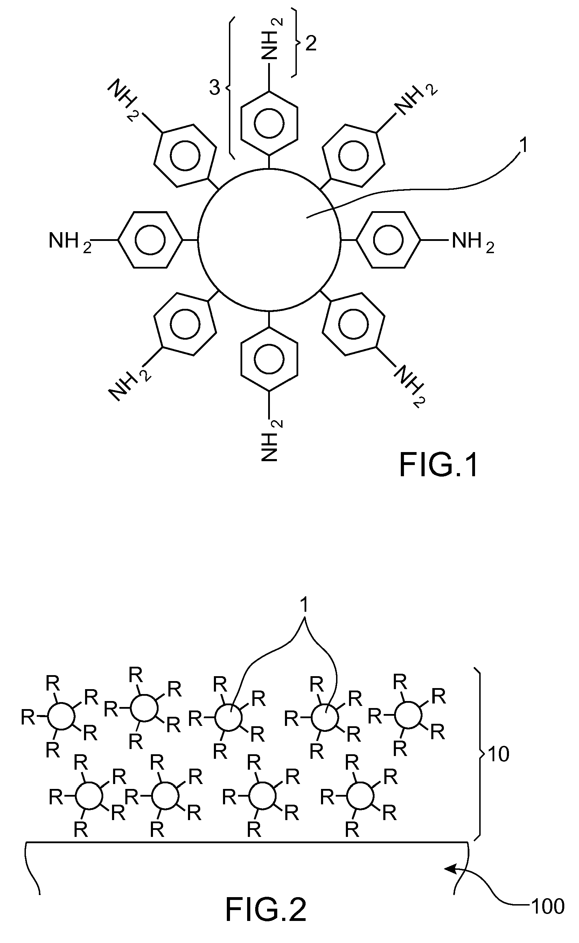

Par exemple, dans la figure 1 est représenté un grain 1 de nano-poudre de diamant greffé par une molécule 3, qui est ici un sel de diazinium. Le groupement fonctionnel 2 de la molécule est ici -NH₂. Cette terminaison aminée peut favoriser la détection de certains composés comportant une fonction acide.

La nano-poudre de diamant, sous sa forme brute, traitée ou greffée, est ensuite déposée sur une face du transducteur acoustique. La couche de nano-poudre peut être déposée selon une couche continue ou selon un motif en des endroits sélectionnés d'une surface du transducteur. Le dépôt peut être réalisé :
- par trempage du transducteur dans une solution contenant la nano-poudre dispersée dans un liquide adéquat ;
- par dépôt à la tournette à partir d'une solution de nano-poudre de diamant dispersée dans un liquide adéquat ;
- par une méthode de pulvérisation adéquate (par exemple par électronébulisation) ;
- par toute autre méthode de dépôt connue de l'homme du métier, et notamment par toute méthode permettant de déposer la nano-poudre sélectivement sur une surface (par exemple par patterning).

A titre d'exemple, la figure 2 représente une vue en coupe d'une couche de nano-poudre de diamant 10, dont les grains 1 sont fonctionnalisés par greffage d'une fonction chimique de reconnaissance R, qui est déposée sur la face supérieure d'un transducteur acoustique 100.

Nous allons à présent décrire en détail plusieurs modes de réalisation d'un capteur du type gravimétrique selon l'invention, en prenant l'exemple d'un capteur SAW.

Selon un premier exemple, on réalise un capteur SAW comprenant une couche de nano-poudre de diamant non traitée. Le capteur ainsi obtenu est dit non spécifique car il n'est pas dédié à la détection d'une espèce chimique ou biochimique particulière.

Dans notre exemple, la nano-poudre de diamant utilisée est la SP3 de chez Seki Technotron, USA. Cette poudre de diamant a une taille moyenne de grains de 50 nanomètres et est dispersée dans une solution d'éthanol pur.

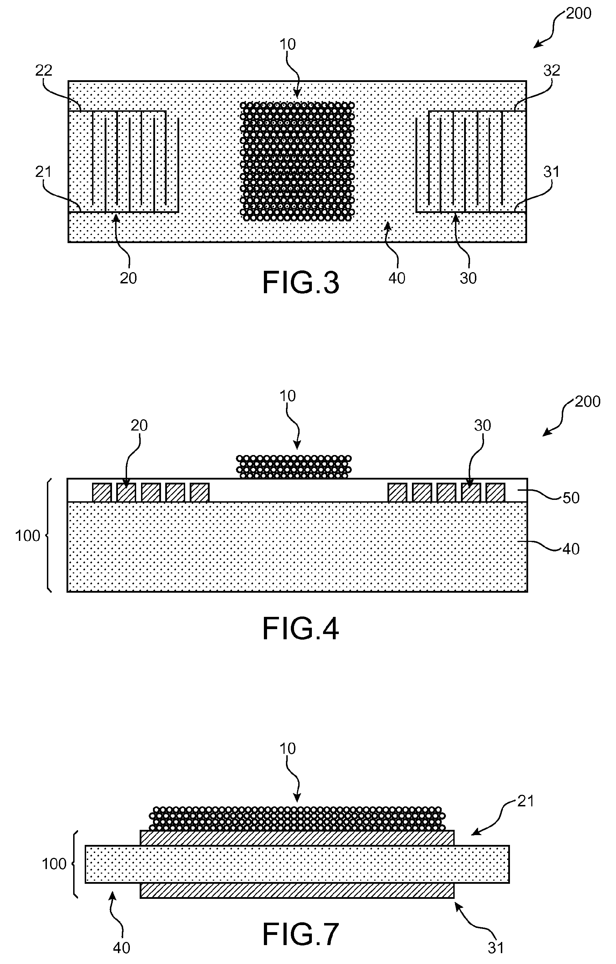

Le transducteur SAW utilisé est un transducteur piézoélectrique 100 comprenant un substrat piézoélectrique 40 en LiTaO₃ sur lequel deux peignes 20, 30 en or, formés par deux électrodes interdigitées (21, 22 et 31, 32), sont déposés sur une même face du substrat piézoélectrique, comme représenté dans la figure 3. Ce transducteur SAW fonctionne avec une fréquence de résonance de 433 MHz.

Le transducteur SAW est lavé par rinçages successifs dans de l'acétone, de l'isopropanol, puis dans de l'eau déionisée, et est ensuite séché sous flux d'argon.

Par la suite, le transducteur SAW est plongé dans une solution de nano-poudre de diamant, obtenue en dispersant la nano-poudre SP3 mentionnée ci-dessus dans une solution d'éthanol non diluée, pendant 3 minutes précisément, puis est immédiatement rincé à l'eau déionisée et enfin, est séché sous flux d'argon.

On obtient ainsi un capteur SAW 200 comprenant une couche sensible 10 en nano-poudre de diamant déposée de façon très homogène sur une face du transducteur SAW 100. Dans la figure 3, la couche sensible 10 est disposée sur la face du substrat piézoélectrique 40 comprenant les deux peignes 20 et 30 et est localisée entre ces deux peignes.

Avantageusement, on peut disposer une couche guidante entre le substrat piézoélectrique et la couche sensible afin, d'une part, d'améliorer les propriétés acoustiques du capteur, et en particulier le couplage de l'onde acoustique se propageant à la surface du transducteur vers la couche sensible, ceci afin de rendre le transducteur plus sensible, et d'autre part, afin de protéger physiquement la surface du transducteur et les électrodes. Dans la figure 4, une couche guidante 50 en SiO₂ est déposée sur la face du matériau piézoélectrique 40 comprenant les deux peignes 20 et 30 de manière à recouvrir complètement cette face et les deux peignes. La couche sensible 10 sera donc déposée sur cette couche guidante 50.

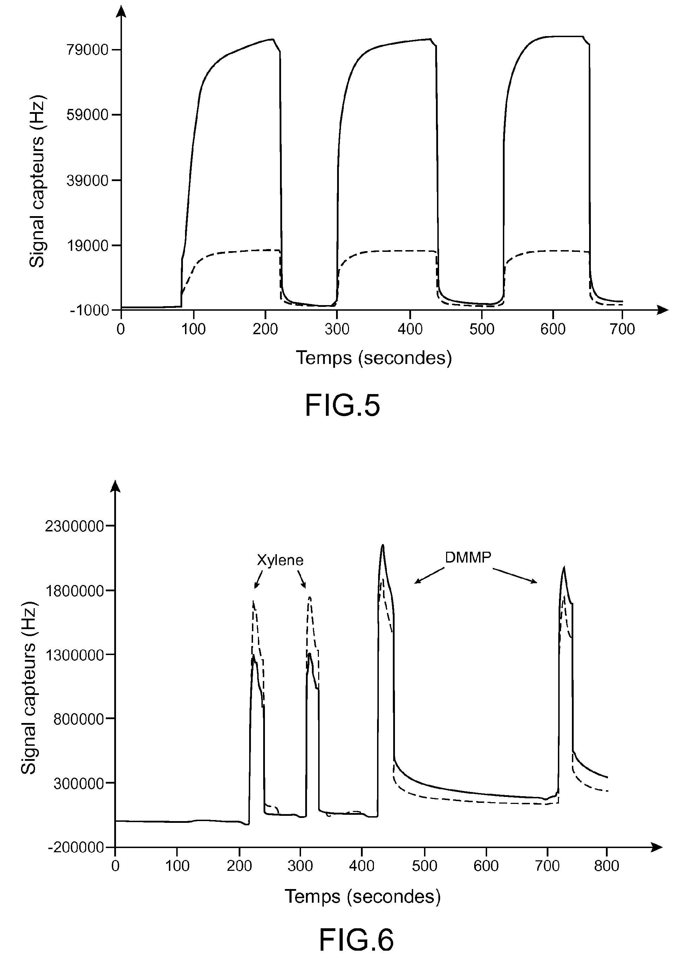

Le capteur SAW obtenu est testé sous vapeurs de xylène et la réponse est comparée à celle obtenue avec un même capteur SAW témoin ne comprenant pas de couche sensible. On mesure les réponses successives de deux capteurs SAW à trois expositions de vapeur de xylène dans l'air synthétique sec (concentration 2500 ppm). Les résultats sont représentés dans la figure 5 : la courbe en pointillés représente la réponse du capteur SAW témoin et la courbe en trait plein représente la réponse du capteur SAW comportant une couche de nano-poudre de diamant non traitée selon l'invention. Comme on peut le constater, la réponse du capteur modifié par l'ajout d'une couche sensible en nano-poudre de diamant est typiquement quatre fois supérieure à celle du capteur SAW témoin, en termes d'amplitude de signal.

Selon un second exemple, on réalise un capteur SAW partiellement spécifique. Pour cela, on utilise une nano-poudre de diamant traitée en surface.

On dit qu'un capteur est partiellement spécifique lorsqu'il est fonctionnalisé de manière à ce que sa fonctionnalisation lui permette d'augmenter sa sensibilité à une ou plusieurs familles de composés en particulier, et non pas à un composé unique. On peut par exemple fonctionnaliser deux capteurs différemment de telle sorte que le premier capteur soit très sensible à une famille de composés A, tandis que le second capteur sera plus sensible à une famille de composés B. Ainsi, lorsque le premier capteur est exposé à un composé de la famille B, ce capteur donne une réponse non négligeable, mais bien plus faible que dans le cas du second capteur.

Les capteurs SAW utilisés sont les mêmes que celui décrit ci-dessus, à savoir un transducteur piézoélectrique comprenant un substrat piézoélectrique en LiTaO₃, sur lequel des peignes interdigités en or sont déposés et qui fonctionne à une fréquence de résonance de 433 MHz.

Deux transducteurs SAW sont lavés par rinçages successifs dans de l'acétone, de l'isopropanol, puis dans de l'eau déionisée. Les deux transducteurs SAW sont ensuite séchés sous flux d'argon.

Le premier transducteur est plongé pendant 3 minutes précisément dans une solution de nano-poudre de diamant obtenue par dispersion de nano-poudre de diamant SP3, de chez Seki Technotron USA, ayant une taille moyenne de grains de 50 nanomètres, dans une solution d'éthanol non diluée.

L'analyse XPS (« X-ray photoemission spectroscopy ») de la nano-poudre SP3 a montré que cette poudre possède une variété de terminaisons de surface (hydroxyle, carboxylique, ponts éther...). Cette poudre possède un potentiel zéta positif de +40 mV.

Le second capteur subit le même traitement avec une nano-poudre de diamant différente, dispersée dans une solution d'éthanol non diluée. On choisit la nano-poudre SYNDIA de Vanmoppes, en Suisse, ayant la référence 11247, grade 0-0.02 GAF, 14,8 carats/kg.

Cette poudre de diamant a été traitée par le fournisseur pour avoir majoritairement en surface des terminaisons carboxyliques (-COOH). Cette solution de nano-poudre possède un potentiel zéta négatif de -35 mV.

Immédiatement après avoir été plongés pendant 3 minutes dans leurs solutions respectives, les deux transducteurs SAW sont rincés à l'eau déionisée, puis séchés sous flux d'argon : on obtient ainsi deux capteurs SAW comprenant une couche sensible à base de nano-poudre de diamant.

Les deux capteurs SAW ainsi obtenus sont testés simultanément pour la détection du xylène, puis du diméthylméthylphosphonate (DMMP). Le résultat est représenté dans la figure 6, où la courbe en trait plein représente le premier capteur SAW et la courbe en traits pointillés représente le second capteur SAW. On constate que les deux capteurs ont des sensibilités différentes : le premier capteur capte plus facilement le DMMP, tandis que le second capteur capte plus facilement le xylène. L'utilisation de deux types de nano-poudres fonctionnalisées avec des groupes fonctionnels différents, et ayant donc des états de surface différents, permet d'inverser l'ordre des sensibilités des deux capteurs aux vapeurs de test et ainsi d'apporter une sélectivité partielle aux capteurs.

Selon un troisième exemple, on réalise un capteur partiellement spécifique, c'est-à-dire un capteur capable de capter plusieurs analytes spécifiques. On utilise pour cela une nano-poudre de diamant greffée en surface par un sel de diazonium.

La nano-poudre de diamant utilisée est par exemple la poudre de détonation SYNDIA de Vanmoppes, Suisse, ayant la référence 11247, grade 0-0.02 GAF, 14,8 carats/kg. Cette poudre possède un potentiel zéta négatif et est soluble dans l'eau. Elle est dispersée dans une solution non diluée d'éthanol.

Le greffage de la nano-poudre est réalisé de la façon suivante : d'une part, 69 mg de NaNO₂ est ajouté à 10 mL de cette solution de nano-poudre (solution 1) ; d'autre part, on prépare 25 mL d'une solution aqueuse de HCl (0,5M) dans laquelle 0,297 mg de 1,4-phénylènediamine est dissout (solution 2). Les deux solutions sont ensuite refroidies à 5 degré Celsius.

La solution 1 est ensuite placée dans un bain de glace et la solution est agitée à l'aide d'un agitateur magnétique. Dans cette solution 1, la solution 2 est versée progressivement, puis le mélange réactionnel est conservé dans le bain de glace sous agitation magnétique pendant une heure. La solution obtenue est finalement ramenée à température ambiante. La solution ainsi obtenue contient une nano-poudre greffée.

La nano-poudre greffée doit à présent être nettoyée. Pour cela, la solution obtenue est centrifugée, puis le précipité de nano-poudre est isolé du liquide et redispersé par ultrasons dans de l'eau distillée. Cette opération est répétée cinq fois. Finalement, la nano-poudre greffée est dispersée par ultrasons une dernière fois dans de l'eau distillée.

Le dépôt de la nano-poudre de diamant fonctionnalisée sur le transducteur SAW est obtenu par trempage du transducteur SAW dans cette solution de nano-poudre, puis séchage du transducteur SAW sous flux d'argon : on obtient alors un capteur SAW comprenant une couche de nano-poudre de diamant fonctionnalisée.

En procédant ainsi, il est possible de greffer de façon covalente des groupes de type phénylamine sur la surface des grains de la nano-poudre de diamant (voir par exemple la figure 1).

Le revêtement obtenu est très riche en groupes fonctionnels de type -NH₂, ce qui favorise l'adsorption, sur la couche sensible du capteur SAW, de composés volatils comportant une fonction acide.

De la même façon, d'autres sels de diazonium pourraient être greffés sur la surface des grains de nano-poudre de diamant. Par exemple, une fonction acide peut être greffée en remplaçant le 1,4-phenylènediamine par de l'acide 4-aminobenzoïque.

Nous venons de décrire des modes de réalisation d'un capteur SAW. Il est cependant possible de réaliser d'autres types de capteurs ayant une couche sensible en nano-poudre de diamant, comme par exemple un transducteur piézoélectrique ou acoustique de type MBQ, micro-levier, FPW, FBAR ou HBAR ou tout autre résonateur acoustique de type gravimétrique. A titre d'exemple, un capteur gravimétrique 200 de type microbalance à quartz MBQ comprenant une couche sensible 10 en nano-poudre de diamant est représenté selon une vue en coupe dans la figure 7 : une couche de matériau piézoélectrique 40 en quartz est prise en sandwich entre une première couche métallique servant de première électrode 21 et une seconde couche métallique servant de seconde électrode 31, et une couche sensible à base de nano-poudre de diamant 10 est disposée sur la première électrode 21.

Un capteur comprenant une couche en nano-poudre de diamant selon l'invention peut être utilisé dans de nombreux domaines, en particulier dans toute application nécessitant la détection chimique ou biochimique, notamment en phase vapeur, que ce soit pour la sécurité anti-terrorisme, le contrôle environnemental ou industriel, où de faibles concentrations de composés chimiques ou biochimique doivent être détectées.

Par exemple, un tel capteur peut être utilisé pour réaliser un nez électronique, capable de détecter la présence d'une ou de plusieurs molécules particulières. Dans cet exemple, plusieurs capteurs sont réalisés en fonctionnalisant des transducteurs acoustiques par l'adjonction de couches sensibles peu sélectives, mais dont la sélectivité varie d'un capteur à l'autre. Chaque capteur comprend une couche de nano-poudre de diamant différente : les groupes fonctionnels greffés sur la nano-poudre de diamant sont différents pour chaque couche sensible. Les capteurs sont ensuite intégrés dans un dispositif comprenant d'une part des moyens permettant à la fois un échantillonnage des produits à détecter ou à analyser, et d'autre part, une électronique d'acquisition simultanée des signaux de chaque capteur. Une analyse multivariable adéquate permet de traiter les données reçues lors de l'exposition à un composé ou à un mélange de composés chimiques afin de reconnaitre une « empreinte » des substances analysées. Un tel dispositif peut servir au contrôle qualité dans l'industrie alimentaire, au contrôle de l'air environnemental ou encore à assister un diagnostique dans le domaine médical.

Il est également possible d'utiliser une couche de nano-poudre de diamant selon l'invention pour concevoir un détecteur de chromatographie en phase gazeuse. Pour cela, de la nano-poudre de diamant non traitée ou ayant subit un traitement d'homogénéisation (oxydation) est déposée sur un transducteur de type SAW. Le capteur obtenu est placé en aval d'une colonne de chromatographie phase gazeuse pour servir de détecteur. Les composés séparés dans la colonne de chromatographie sont détectés séquentiellement en sortie de colonne. Le phénomène de détection repose sur l'adsorption non spécifique (la nano-poudre n'étant pas fonctionnalisée) des molécules sur la nano-poudre. La nano-poudre offre une surface d'adsorption accrue et permet donc d'augmenter la sensibilité du détecteur tout en ayant un coefficient de diffusion élevé pour permettre une réponse rapide du détecteur.

Selon un troisième exemple d'application, on peut utiliser une couche sensible de nano-poudre de diamant pour réaliser un détecteur portable permettant de détecter des produits sous forme de traces. Pour cela, une couche de nano-poudre de diamant fonctionnalisée par une fonction de reconnaissance très spécifique (par exemple pour un couplage corps-anticorps) est déposée sur un transducteur acoustique. Le capteur obtenu est placé dans un détecteur portable pour la détection de produits spécifiques à l'état de trace. Un tel dispositif peut être un détecteur de fuite sur une ligne de gaz, un outil de protection personnelle face à une exposition à un composé très toxique (par exemple un agent neurotoxique de guerre) ou un détecteur de substances illicites (cocaïne...).

Les avantages de la nano-poudre de diamant par rapport aux couches sensibles utilisées dans l'art antérieur pour réaliser des couches sensibles destinées à être déposées sur des transducteur acoustiques sont multiples. Elle permet en particulier :
- de faciliter le dépôt de couches sensibles homogènes, donnant une réponse plus reproductible d'un capteur à l'autre ;
- d'augmenter la surface active d'adsorption des molécules à détecter et ainsi d'augmenter la sensibilité du capteur ;
- de limiter les paramètres autres que la masse pouvant affecter les propriétés acoustiques de la couche sensible par rapport à d'autres couches sensibles de l'art antérieur ;
- de permettre le greffage covalent à la surface de la nano-poudre de diamant (constituée de carbone) d'un grand nombre de groupes fonctionnels choisis pour leur grande affinité avec les molécules à détecter.

En utilisant de la nano-poudre de diamant, il est possible de contrôler parfaitement l'état de surface de la couche sensible sur laquelle les espèces chimiques ou biochimiques seront piégées. Il est également possible de fonctionnaliser la couche sensible pour augmenter sa sélectivité. Cela est possible car, comme la couche sensible est réalisée à partir d'une poudre de diamant ayant des grains de tailles nanométriques, la surface et l'épaisseur de la couche sensible sont uniformes, d'une part, et d'autre part, la surface de la couche sensible est constituée essentiellement d'atomes de carbone. Or, la chimie du carbone offre de nombreuses possibilités de greffage de groupements chimiques ou biologiques divers. Il est donc aisé de greffer des groupes fonctionnels sur la surface de la nano-poudre de diamant.

La couche sensible en nano-poudre de diamant selon l'invention est homogène. En effet, comme les grains de la poudre de diamant sont de tailles nanométriques, le rapport surface / volume des grains de la poudre est élevé, ce qui a pour conséquence que les grains viennent s'adhérer spontanément sur les surfaces rencontrées, majoritairement par forces de Van Der Waals. Ainsi, une méthode simple pour déposer la nano-poudre sur la surface d'un transducteur est de plonger le transducteur dans une solution de nano-poudre bien dispersée. La quantité de nano-poudre déposée sera alors fonction du temps de plongée dans la solution.

D'autres méthodes de dépôt, qui ne nécessitent pas l'ajout d'additifs dans la solution de nano-poudre (comme par exemple des surfactants), peuvent également être utilisées, comme par exemple le dépôt par tournette d'une solution dispersée de nano-poudre ou le dépôt par électronébulisation. La condition nécessaire et suffisante à l'obtention d'un dépôt uniforme est la dispersion uniforme de la nano-poudre de diamant dans la solution de préparation.

Les méthodes pour disperser de la nano-poudre de diamant brute ou oxydée dans de l'eau ou de l'éthanol sont connues. Si la nano-poudre de diamant est fonctionnalisée par des fonctions de reconnaissance spécifique, il est possible que la poudre soit plus difficile à disperser. Cependant, de part leur géométrie quasi-sphérique, les grains de la nano-poudre de diamant restent plus faciles à disperser en solution que, par exemple, les nano tubes de carbone de l'art antérieur.

L'utilisation de couches sensibles sur des transducteurs acoustiques nécessite un contrôle strict des propriétés de la couche sensible et notamment l'épaisseur, l'uniformité et adhérence de la couche sensible, afin d'obtenir des performances suffisantes et reproductibles. La nano-poudre de diamant présente l'avantage de pouvoir se déposer en couches minces de façon très homogène et reproductible sur un grand nombre de surfaces solides, et en particulier sur les surfaces constituant les transducteurs acoustiques, à savoir les métaux utilisés pour fabriquer les électrodes des transducteurs (généralement en or, en platine ou en aluminium), ainsi que les matériaux piézoélectriques constituant le transducteur (oxyde de zinc (ZnO), nitrure d'aluminium (AlN), quartz, tantalate de lithium (LiTaO₃), niobate de lithium (LiNbO₃)) ou certaines couches de guide d'onde déposées sur les transducteurs pour améliorer leurs performances acoustiques.

D'autre part, une couche en nano-poudre de diamant offre une surface active très importante. Ceci permet d'offrir plus de sites d'interactions avec les espèces chimiques ou biochimiques à détecter et donc une meilleure sensibilité du capteur. Le coefficient de diffusion à travers la couche est aussi augmenté par rapport à des couches sensibles d'autres natures, par exemple des films polymères. Le temps de réponse des capteurs est par conséquent significativement diminué.

Par ailleurs, la nano-poudre de diamant est chimiquement inerte. De ce fait, la stabilité sur le long terme de la couche sensible est améliorée par rapport, par exemple, à certains films polymères pouvant subir un vieillissement bien connu de l'homme du métier. Ainsi, la durée de vie du capteur est généralement augmentée et sa dérive dans le temps est diminuée. Lorsque la nano-poudre de diamant est fonctionnalisée, il est possible d'observer un vieillissement du fait de l'altération, dans le temps, de la surface greffée. Néanmoins, le greffage sera généralement fait par liaisons covalentes qui, pour certaines, sont réputées être très stables dans le temps.

Un autre avantage de l'invention particulier aux transducteurs acoustiques dont le fonctionnement est basé sur les ondes de surface, comme les transducteurs SAW, tient au mode de fonctionnement de ces capteurs. En effet, lors de l'utilisation d'un transducteur acoustique dont le fonctionnement est basé sur les ondes de surface, toute variation des propriétés acoustiques à la surface du capteur, notamment les variations dues aux paramètres intrinsèques du matériau de surface du capteur, contribuent à faire varier la fréquence de résonance du capteur. Le fait que la couche sensible soit en nano-poudre de diamant apporte de nombreux avantages par rapport à l'art antérieur car :
- d'une part, lorsque la nano-poudre de diamant n'est pas fonctionnalisée, car elle est alors intrinsèquement isolante. Ainsi, contrairement aux nanotubes de carbone par exemple, qui, de part la configuration SP2 des atomes de carbone les constituant, sont intrinsèquement conducteurs ou semiconducteurs, les effets électroacoustiques à la surface du transducteur deviennent négligeables lorsque la nano-poudre de diamant est utilisée comme couche sensible. Ainsi, seule l'augmentation de la masse de la couche sensible, provenant de l'adsorption d'espèces chimiques ou biochimiques sur la couche sensible, fait varier la fréquence de résonance du transducteur.
- d'autre part, la nature physique de la nano-poudre de diamant, en particulier l'absence de chaines polymères dans la couche sensible, ainsi que la géométrie quasi sphérique (en opposition aux nano tubes de carbone), rend la couche sensible très peu élastique. Les effets viscoélastiques à la surface des transducteurs sont donc eux aussi réduits.

Par conséquent, l'utilisation de nano-poudre de diamant permet de s'affranchir de plusieurs paramètres acoustiques pouvant modifier la fréquence de résonance du transducteur. Ainsi, l'utilisation de nano-poudre de diamant permet au transducteur acoustique de tendre vers un régime gravimétrique, où seule l'augmentation de la masse de la couche sensible, provenant de l'adsorption d'espèces chimiques ou biochimiques sur la couche sensible, fait varier la fréquence de résonance du transducteur. Ceci permet d'améliorer la stabilité et la fiabilité du capteur, et potentiellement à améliorer le rapport signal/bruit du capteur.

### BIBLIOGRAPHIE

**[1]** "Polymer Films in Sensor Applications: Technology, Materials, Devices and their Characteristics", G. Harsanyl, Technomics Publishing Co., Lancaster, Basel, 1995, p. 435.
**[2]** "Acoustic wave microsensor arrays for vapor sensing", J.W. Grate , Chem. Rev., 100, (2000), p. 2627-2648.
**[3]** "Dewetting effect on polymer-coated surface acoustic wave vapor sensors", J.W. Grate et R.A. McGill , Anal. Chem., 67, (1995), p. 4015-4019.
**[4]** "Gas phase detection of explosives such as 2,4,6-trinitrotoluene by molecularly imprinted polymers", Gudrun Bunte et al., Analytica Chimica Acta, 591, (2007), p. 49-56.
**[5]** "Enhanced sensitivity of SAW gas sensor coated molecularly imprinted polymer incorporating high frequency stability oscillator", Wang Wen et al., Sensors and Actuators, B, 125, (2007), p. 422-427.
**[6]** "The application of metalloporphyrins as coating material for quartz microbalance-based chemical sensors", J.A.J. Brunink et al., Analytica Chimica Acta, 325, (1996), p. 53-64.
**[7]** "Thickness Dependence of the Optical Anisotropy for Porphyrin Octaester Langmuir-Schaefer Films", C. Goletti et al., Langmuir, 2002, 18, p. 6881-6886.
**[8]** "Layered SAW gas sensor with single-walled carbon nanotube-based nanocomposite coating", M. Penza et al., Sensors and Actuators, B, 127, (2007), p. 168-178
**[9]** "Bilayer structure for hydrogen detection in a surface acoustic wave sensor system", W.P. Jakubik et al., Sens. Actuators, B, 82, (2002), p. 265-271.
**[10]** EP 1 926 211, déposé le 20 novembre 2007.

## Revendications

1. Capteur chimique ou biochimique gravimétrique du type résonateur acoustique (200), comprenant :
- un transducteur gravimétrique du type résonateur acoustique (100) ;
- une couche, appelée couche sensible (10), apte à adsorber une ou plusieurs espèces chimiques ou biochimiques déterminées, ladite couche sensible étant formée sur une face du transducteur gravimétrique du type résonateur acoustique,
**caractérisé en ce que** la couche sensible (10) comprend une poudre de diamant dont les grains (1) sont de taille nanométrique et forment un empilement tridimensionnel.

2. Capteur chimique ou biochimique gravimétrique du type résonateur acoustique selon la revendication 1, dans lequel la couche sensible (10) est constituée uniquement de la poudre de diamant dont les grains (1) sont de taille nanométrique.

3. Capteur chimique ou biochimique gravimétrique du type résonateur acoustique selon la revendication 1 ou 2, dans lequel le transducteur gravimétrique du type résonateur acoustique est un transducteur piézoélectrique.

4. Capteur chimique ou biochimique gravimétrique du type résonateur acoustique selon la revendication 3, dans lequel le transducteur piézoélectrique (100) est un transducteur à onde acoustique de surface (SAW).

5. Capteur chimique ou biochimique gravimétrique du type résonateur acoustique selon la revendication 3, dans lequel le transducteur piézoélectrique (100) est un transducteur à microbalance à quartz (MBQ).

6. Capteur chimique ou biochimique gravimétrique du type résonateur acoustique selon la revendication 3, dans lequel le transducteur piézoélectrique est un transducteur choisi parmi un transducteur à ondes de flexion (FPW), un transducteur à ondes acoustiques de volume (FBAR) ou un transducteur à onde acoustique de volume en mode harmonique élevé (HBAR).

7. Capteur chimique ou biochimique gravimétrique du type résonateur acoustique selon la revendication 1 ou 2, dans lequel le transducteur gravimétrique du type résonateur acoustique est un transducteur à micro-levier.

8. Capteur chimique ou biochimique gravimétrique du type résonateur acoustique selon la revendication 1 ou 2, dans lequel les grains (1) de la poudre de diamant ont un diamètre compris entre 1 nm et 200 nm.

9. Capteur chimique ou biochimique gravimétrique du type résonateur acoustique selon la revendication 8, dans lequel les grains (1) de la poudre de diamant ont un diamètre compris entre 1 nm et 50 nm.

10. Capteur chimique ou biochimique gravimétrique du type résonateur acoustique selon la revendication 8, dans lequel les grains (1) de la poudre de diamant ont un diamètre compris entre 1 nm et 15 nm.

11. Capteur chimique ou biochimique gravimétrique du type résonateur acoustique selon la revendication 1 ou 2, dans lequel les grains (1) de la poudre de diamant sont oxydés en surface.

12. Capteur chimique ou biochimique gravimétrique du type résonateur acoustique selon la revendication 1 ou 2, dans lequel des molécules (3) ayant des groupements fonctionnels (2) aptes à réagir spécifiquement avec un groupement fonctionnel des espèces chimiques ou biochimiques devant être captées sont greffées en surface des grains (1) de la poudre de diamant.

13. Capteur chimique ou biochimique gravimétrique du type résonateur acoustique selon la revendication 12, dans lequel les molécules (3) greffées en surface des grains (1) de la poudre de diamant sont des macromolécules choisies parmi les porphyrines, les phtalocyanines, les corroles ou des complexes métalliques de celles-ci.

14. Dispositif de détection d'espèces chimiques ou biochimiques comprenant au moins deux capteurs chimiques ou biochimiques gravimétriques du type résonateur acoustique (200) tels que définis dans la revendication 12 ou 13, dans lequel les groupements fonctionnels (2) des molécules (3) greffées en surface des grains (1) de la poudre de diamant d'une première couche sensible sont différents des groupements fonctionnels des molécules greffées en surface des grains de la poudre de diamant d'une seconde couche sensible parmi les couches sensibles desdits au moins deux capteurs chimiques ou biochimiques gravimétriques du type résonateur acoustique.

15. Utilisation d'une couche de poudre de diamant dont les grains (1) sont de taille nanométrique comme couche sensible (10) à déposer sur un transducteur gravimétrique du type résonateur acoustique (100) pour former un capteur chimique ou biochimique gravimétrique du type résonateur acoustique (200) selon l'une quelconque des revendications 1 à 13.

16. Utilisation d'une couche de poudre de diamant selon la revendication 15, dans laquelle la taille des grains (1) est comprise entre 1 nm et 200 nm.

17. Procédé de réalisation d'un capteur chimique ou biochimique gravimétrique du type résonateur acoustique (200) tel que défini dans l'une quelconque des revendications 1 à 13, comprenant les étapes suivantes :
a) fourniture d'une solution comprenant de la poudre de diamant dispersée, dont les grains sont de taille nanométrique ;
b) formation d'une couche de poudre de diamant sur une face du transducteur gravimétrique de type résonateur acoustique en utilisant la solution fournie à l'étape a).

18. Procédé de réalisation d'un capteur chimique ou biochimique selon la revendication 17, dans lequel la solution de l'étape a) est obtenue en dispersant de la poudre de diamant dont les grains sont de taille nanométrique dans de l'eau ou de l'éthanol.

19. Procédé de réalisation d'un capteur chimique ou biochimique selon la revendication 17, dans lequel la solution de l'étape a) est obtenue en oxydant la surface des grains d'une poudre de diamant, les grains étant de taille nanométrique, et en dispersant lesdits grains dans de l'eau ou de l'éthanol.

20. Procédé de réalisation d'un capteur chimique ou biochimique selon la revendication 17, dans lequel la solution de l'étape a) est obtenue en réalisant les étapes suivantes :
- oxydation de la surface des grains d'une poudre de diamant, les grains étant de taille nanométrique ;
- formation d'une solution par dispersion desdits grains oxydés dans un solvant adéquat, préférentiellement de l'eau ou de l'éthanol ;
- ajout, dans la solution, de réactifs chimiques permettant le greffage préférentiellement covalent de groupements fonctionnels sur les grains oxydés de la poudre de diamant.

21. Procédé de réalisation d'un capteur chimique ou biochimique selon la revendication 17, dans lequel la solution de l'étape a) est obtenue en réalisant les étapes suivantes :
- hydrogénation de la surface des grains d'une poudre de diamant, les grains étant de taille nanométrique ;
- formation d'une solution par mise en solution desdits grains hydrogénés dans un solvant adéquat pour les réactions futures de greffage ;
- ajout, dans la solution, de réactifs chimiques permettant le greffage préférentiellement covalent de groupements fonctionnels sur les grains hydrogénés de la poudre de diamant, et le greffage permettant une dispersion des grains initialement hydrogénés dans la solution.

22. Procédé de réalisation d'un capteur chimique ou biochimique selon l'une quelconque des revendications 17 à 21, dans lequel la taille des grains (1) est comprise entre 1 nm et 200 nm.

## Patentansprüche

1. Gravimetrischer chemischer oder biochemischer Sensor vom Typ akustischer Resonator (200), umfassend:
- einen gravimetrischen Wandler vom Typ akustischer Resonator (100);
- eine Schicht, genannt sensitive Schicht (10), die dazu ausgelegt ist, ein oder mehrere bestimmte chemische oder biochemische Sorten zu adsorbieren, wobei die sensitive Schicht auf einer Fläche des gravimetrischen Wandlers vom Typ akustischer Resonator gebildet ist,
**dadurch gekennzeichnet, dass** die sensitive Schicht (10) ein Diamantpulver umfasst, dessen Körner (1) von nanometrischer Größe sind und einen dreidimensionalen Stapel bilden.

2. Gravimetrischer chemischer oder biochemischer Sensor vom Typ akustischer Resonator nach Anspruch 1, bei dem die sensitive Schicht (10) ausschließlich aus dem Diamantpulver gebildet ist, dessen Körner (1) von nanometrischer Größe sind.

3. Gravimetrischer chemischer oder biochemischer Sensor vom Typ akustischer Resonator nach Anspruch 1 oder 2, bei dem der gravimetrische Wandler vom Typ akustischer Resonator ein piezoelektrischer Wandler ist.

4. Gravimetrischer chemischer oder biochemischer Sensor vom Typ akustischer Resonator nach Anspruch 3, bei dem der piezoelektrische Wandler (100) ein Oberflächenschallwellen-(SAW-) Wandler ist.

5. Gravimetrischer chemischer oder biochemischer Sensor vom Typ akustischer Resonator nach Anspruch 3, bei dem der piezoelektrische Wandler (100) ein Quartzmikrowaage-(MBQ-) Wandler ist.

6. Gravimetrischer chemischer oder biochemischer Sensor vom Typ akustischer Resonator nach Anspruch 3, bei dem der piezoelektrische Wandler ein Wandler ist, ausgewählt aus einem Beugungswellen-(FPW-) Wandler, einem Volumenschallwellen- (FBAR) Wandler oder einem Volumenschallwellen-Wandler in höherem harmonischem Modus (HBAR).

7. Gravimetrischer chemischer oder biochemischer Sensor vom Typ akustischer Resonator nach Anspruch 1 oder 2, bei dem der gravimetrische Wandler vom Typ akustischer Resonator ein Mikrohebel-Wandler ist.

8. Gravimetrischer chemischer oder biochemischer Sensor vom Typ akustischer Resonator nach Anspruch 1 oder 2, bei dem die Körner (1) des Diamantpulvers einen Durchmesser aufweisen, der zwischen 1 nm und 200 nm enthalten ist.

9. Gravimetrischer chemischer oder biochemischer Sensor vom Typ akustischer Resonator nach Anspruch 8, bei dem die Körner (1) des Diamantpulvers einen Durchmesser aufweisen, der zwischen 1 nm und 50 nm enthalten ist.

10. Gravimetrischer chemischer oder biochemischer Sensor vom Typ akustischer Resonator nach Anspruch 8, bei dem die Körner (1) des Diamantpulvers einen Durchmesser aufweisen, der zwischen 1 nm und 15 nm enthalten ist.

11. Gravimetrischer chemischer oder biochemischer Sensor vom Typ akustischer Resonator nach Anspruch 1 oder 2, bei dem die Körner (1) des Diamantpulvers an der Oberfläche oxidiert sind.

12. Gravimetrischer chemischer oder biochemischer Sensor vom Typ akustischer Resonator nach Anspruch 1 oder 2, bei dem an der Oberfläche der Körner (1) des Diamantpulvers Moleküle (3) gepfropft sind, die funktionelle Gruppen (2) haben, welche dazu ausgelegt sind, spezifisch mit einer funktionellen Gruppe der chemischen oder biochemischen Sorten zu reagieren, die erfasst werden sollen.

13. Gravimetrischer chemischer oder biochemischer Sensor vom Typ akustischer Resonator nach Anspruch 12, bei dem die Moleküle (3), die an die Oberfläche der Körner (1) des Diamantpulvers gepfropft sind, Makromoleküle sind, die ausgewählt sind aus den Porphyrinen, den Phtalocyaninen, den Corrolen oder metallischen Komplexen derselben.

14. Vorrichtung zur Erfassung von chemischen oder biochemischen Sorten, umfassend wenigstens zwei gravimetrische chemische oder biochemische Sensoren vom Typ akustischer Resonator (200) wie in Anspruch 12 oder 13 definiert, wobei die funktionellen Gruppen (2) der Moleküle (3), die an die Oberfläche der Körner (1) des Diamantpulvers einer ersten sensitiven Schicht gepfropft sind, verschieden sind von den funktionellen Gruppen der Moleküle, die an die Oberfläche der Körner des Diamantpulvers einer zweiten sensitiven Schicht aus den sensitiven Schichten der wenigstens zwei gravimetrischen chemischen oder biochemischen Sensoren vom Typ akustischer Resonator gepfropft sind.

15. Verwendung einer Schicht aus Diamantpulver, dessen Körner (1) von nanometrischer Größe sind, als sensitive Schicht (10) zur Aufbringung auf einem gravimetrischen Wandler vom Typ akustischer Resonator (100), um einen gravimetrischen chemischen oder biochemischen Sensor vom Typ akustischer Resonator (200) nach einem der Ansprüche 1 bis 13 zu bilden.

16. Verwendung einer Schicht von Diamantpulver nach Anspruch 15, bei der die Größe der Körner (1) zwischen 1 nm und 200 nm enthalten ist.

17. Verfahren zur Herstellung eines gravimetrischen chemischen oder biochemischen Sensors vom Typ akustischer Resonator (200) wie in einem der Ansprüche 1 bis 13 definiert, umfassend die folgenden Schritte:
a) Bereitstellen einer Lösung, die dispergiertes Diamantpulver enthält, dessen Körner von nanometrischer Größe sind;
b) Bilden einer Schicht von Diamantpulver auf einer Fläche des gravimetrischen Wandlers vom Typ akustischer Resonator unter Verwendung der im Schritt a) bereitgestellten Lösung.

18. Verfahren zur Herstellung eines chemischen oder biochemischen Sensors nach Anspruch 17, bei dem die Lösung des Schritts a) erhalten wird durch Dispergieren des Diamantpulvers, dessen Körner von nanometrischer Größe sind, in Wasser oder Ethanol.

19. Verfahren zur Herstellung eines chemischen oder biochemischen Sensors nach Anspruch 17, bei dem die Lösung des Schritts a) erhalten wird durch Oxidieren der Oberfläche der Körner eines Diamantpulvers, wobei die Körner von nanometrischer Größe sind, und durch Dispergieren der Körner in Wasser oder Ethanol.

20. Verfahren zur Herstellung eines chemischen oder biochemischen Sensors nach Anspruch 17, bei dem die Lösung des Schritts a) erhalten wird durch Ausführen der nachfolgenden Schritte:
- Oxidieren der Oberfläche der Körner eines Diamantpulvers, wobei die Körner von nanometrischer Größe sind;
- Bilden einer Lösung durch Dispergieren der oxidierten Körner in einem adäquaten Lösungsmittel, vorzugsweise Wasser oder Ethanol;
- Hinzufügen von chemischen Reagenzien zu der Lösung, die das vorzugsweise kovalente Pfropfen von funktionellen Gruppen auf den oxidierten Körnern des Diamantpulvers ermöglichen.

21. Verfahren zur Herstellung eines chemischen oder biochemischen Sensors nach Anspruch 17, bei dem die Lösung des Schritts a) erhalten wird durch Ausführen der nachfolgenden Schritte:
- Hydrogenieren der Oberfläche der Körner eines Diamantpulvers, wobei die Körner von nanometrischer Größe sind;
- Bilden einer Lösung durch in Lösung bringen der hydrogenierten Körner in einem adäquaten Lösungsmittel für die zukünftigen Pfropfreaktionen;
- Hinzufügen von chemischen Reagenzien zu der Lösung, die das vorzugsweise kovalente Pfropfen von funktionellen Gruppen auf den hydrogenierten Körnern des Diamantpulvers ermöglichen, und wobei das Pfropfen eine Dispersion der anfänglich hydrogenierten Körner in der Lösung ermöglicht.

22. Verfahren zur Herstellung eines chemischen oder biochemischen Sensors nach einem der Ansprüche 17 bis 21, bei dem die Größe der Körner (1) zwischen 1 nm und 200 nm enthalten ist.

## Claims

1. A chemical or biochemical gravimetric sensor of acoustic resonator type (200) comprising:
- a gravimetric transducer of acoustic resonator type (100);
- a layer called sensitive layer (10) capable of adsorbing one or more determined chemical or biochemical species, said sensitive layer being formed on one surface of the gravimetric transducer of acoustic resonator type,
**characterized in that** the sensitive layer (10) comprises diamond powder having particles (1) of nanometric size forming a three-dimensional stack.

2. The chemical or biochemical gravimetric sensor of acoustic resonator type according to claim 1, wherein the sensitive layer (10) is formed solely of diamond powder having particles (1) of nanometric size.

3. The chemical or biochemical gravimetric sensor of acoustic resonator type according to claim 1 or 2, wherein the gravimetric transducer of acoustic resonator type is a piezoelectric transducer.

4. The chemical or biochemical gravimetric sensor of acoustic resonator type according to claim 3, wherein the piezoelectric transducer (100) is a surface acoustic wave transducer (SAW).

5. The chemical or biochemical gravimetric sensor of acoustic resonator type according to claim 3, wherein the piezoelectric transducer (100) is a quartz crystal microbalance transducer (QMB).

6. The chemical or biochemical gravimetric sensor of acoustic resonator type according to claim 3, wherein the piezoelectric transducer is a transducer selected from among a flexural plate wave transducer (FPW), a film bulk acoustic resonator transducer (FBAR) or a high-overtone bulk acoustic resonator transducer (HBAR).

7. The chemical or biochemical gravimetric sensor of acoustic resonator type according to claim 1 or 2, wherein the gravimetric transducer of acoustic resonator type is a micro-lever transducer.

8. The chemical or biochemical gravimetric sensor of acoustic resonator type according to claim 1 or 2, wherein the particles (1) of diamond powder have a diameter between 1 nm and 200 nm.

9. The chemical or biochemical gravimetric sensor of acoustic resonator type according to claim 8, wherein the particles (1) of diamond powder have a diameter between 1 nm and 50 nm.

10. The chemical or biochemical gravimetric sensor of acoustic resonator type according to claim 8, wherein the particles (1) of diamond powder have a diameter between 1 nm and 15 nm.

11. The chemical or biochemical gravimetric sensor of acoustic resonator type according to claim 1 or 2, wherein the particles (1) of diamond powder are surface oxidized.

12. The chemical or biochemical gravimetric sensor of acoustic resonator type according to claim 1 or 2, wherein molecules (3) having functional groups (2) able to react specifically with a functional group of the chemical or biochemical species to be sensed are grafted on the surface of the particles (1) of diamond powder.

13. The chemical or biochemical gravimetric sensor of acoustic resonator type according to claim 12, wherein the molecules (3) grafted on the surface of the particles (1) of diamond powder are macromolecules selected from among porphyrins, phthalocyanines, corroles or metal complexes thereof.

14. A device to detect chemical or biochemical species comprising at least two chemical or biochemical gravimetric sensors of acoustic resonator type (200) such as defined in claim 12 or 13, wherein the functional groups (2) of the molecules (3) grafted on the surface of the particles (1) of diamond powder of a first sensitive layer differ from the functional groups of the molecules grafted on the surface of the diamond powder particles of a second sensitive layer among the sensitive layers of said at least two chemical or biochemical gravimetric sensors of acoustic resonator type.

15. Use of a layer of diamond powder having particles (1) of nanometric size as sensitive layer (10) to be deposited on a gravimetric transducer of acoustic resonator type (100) to form a chemical or biochemical gravimetric sensor of acoustic resonator type (200) according to any of claims 1 to 13.

16. The use of a layer of diamond powder according to claim 15, wherein the size of the particles (1) is between 1 nm and 200 nm.

17. A process to produce a chemical or biochemical gravimetric sensor of acoustic resonator type (200) such as defined in any of claims 1 to 13, comprising the following steps:
a) providing a solution containing dispersed diamond powder having particles of nanometric size;
b) forming a layer of diamond powder on one surface of the gravimetric transducer of acoustic resonator type using the solution provided at step a).

18. The process to produce a chemical or biochemical sensor according to claim 17, wherein the solution of step a) is obtained by dispersing diamond powder having particles of nanometric size in water or ethanol.

19. The process to produce a chemical or biochemical sensor according to claim 17, wherein the solution of step a) is obtained by oxidizing the surface of the particles of a diamond powder, the particles being of nanometric size, and dispersing said particles in water or ethanol.

20. The process to produce a chemical or biochemical sensor according to claim 17, wherein the solution of step a) is obtained by performing the following steps:
- oxidizing the surface of the particles of a diamond powder, the particles being of nanometric size;
- forming a solution by dispersing said oxidized particles in a suitable solvent, preferably water or ethanol;
- adding chemical reagents to the solution allowing the preferably covalent grafting of functional groups onto the oxidized particles of diamond powder.

21. The process to produce a chemical or biochemical sensor according to claim 17, wherein the solution of step a) is obtained by performing the following steps:
- hydrogenating the surface of the particles of a diamond powder, the articles being of nanometric size;
- forming a solution by solubilising said hydrogenated particles in a suitable solvent for subsequent grafting reactions;
- adding chemical reagents to the solution allowing preferably covalent grafting of functional groups onto the hydrogenated particles of diamond powder, and grafting to allow dispersion of the initially hydrogenated particles in the solution.

22. The process to produce a chemical or biochemical sensor according to any of claims 17 to 21, wherein the size of the particles (1) is between 1 nm and 200 nm.
